# EUROPEAN PATENT SPECIFICATION

(11) **EP 3 515 316 B1**
(45) Date of publication and mention of the grant of the patent: **25.11.2026**
(21) Application number: 17853339.4
(22) Date of filing: 12.09.2017
(51) Int. Cl.: A61B 8/00, A61G 15/00, A61G 15/02, A61B 5/00, A61G 13/00, A61B 8/08, A61B 8/14, A61B 5/01, A61B 5/103

(54) **OBSTETRIC AND GYNECOLOGIC DIAGNOSIS APPARATUS AND OBSTETRIC AND GYNECOLOGIC DIAGNOSIS METHOD USING THE SAME**
VORRICHTUNG ZUR GEBURTSHILFE- UND GYNÄKOLOGISCHEN DIAGNOSE UND VERFAHREN ZUR GEBURTSHILFE- UND GYNÄKOLOGISCHEN DIAGNOSE DAMIT
APPAREIL DE DIAGNOSTIC OBSTÉTRIQUE ET GYNÉCOLOGIQUE ET PROCÉDÉ DE DIAGNOSTIC OBSTÉTRIQUE ET GYNÉCOLOGIQUE L'UTILISANT

(30) Priority: 23.09.2016 US 201662398689 P; 25.07.2017 KR 20170094320
(43) Date of publication of application: 31.07.2019
(73) Proprietor: Samsung Medison Co., Ltd., Hongcheon-gun, Gangwon-do 25108 (KR)
(72) Inventor: LIM, Sung-won, Hongcheon-gun Gangwon-do 25108 (KR); KIM, Ui, Hongcheon-gun Gangwon-do 25108 (KR); MOON, Jun-pil, Hongcheon-gun Gangwon-do 25108 (KR); SHIN, Cheon-seop, Hongcheon-gun Gangwon-do 25108 (KR); HA, Kil-su, Hongcheon-gun Gangwon-do 25108 (KR)
(74) Representative: Arnold & Siedsma
(86) International application number: PCT/KR2017/009966
(87) International publication number: WO 2018/056625

(56) References cited:
- WO-A1-2013/112107
- CN-U- 201 572 283
- JP-A- H08 684
- KR-A- 20050 119 921
- KR-A- 20150 052 673

## Description

### [Technical Field]

The present disclosure relates to an obstetric and gynecologic diagnosis apparatus including an ultrasound probe for providing an ultrasound image and an obstetric and gynecologic chair part, and a method using the obstetric and gynecologic diagnosis apparatus.

### [Background Art]

Ultrasound diagnosis apparatuses transmit an ultrasound signal generated by a transducer of a probe to an object and receive information regarding a signal reflected from the object, thereby obtaining an image of a part (e.g., a soft tissue or a blood stream) inside the object by using a noninvasive method.

Ultrasound diagnosis apparatuses are small and inexpensive, display images in real time, and are safe with respect to a fetus due to the lack of radioactive exposure, compared to other image diagnosis apparatuses, such as X-ray diagnosis apparatuses, computerized tomography (CT) scanners, magnetic resonance imaging (MRI) apparatuses, and nuclear medical diagnosis apparatuses. Therefore, ultrasound diagnosis apparatuses are widely used for obstetric and gynecologic diagnosis.

In the case of a mother who is carrying a fetus, her external appearance rapidly changes according to the growth of the fetus, and diagnostic items also change according to the growth of the fetus. According to the changes in the external appearance of the mother and diagnostic items, the diagnostic posture of the mother changes, and accordingly, the shape of a chair for obstetric and gynecologic diagnosis, on which the mother is seated to receive an obstetric or gynecologic diagnosis, should be changed.

In accordance with its abstract, patent application document KR 2015 0 052 673 A describes that an integrated chair unit for the obstetric and gynecologic diagnosis according to an embodiment comprises: a chair unit which holds an object, has a length in a first direction and includes a holder capable of holding legs of the object; and a diagnosis unit which diagnoses the object and is connected to the chair unit to be arranged on a side of a second direction which crosses the first direction. The integrated chair unit for the obstetric and gynecologic diagnosis improves the convenience of a patient or a user and minimizes a diagnosis time and the sense of shame and anxiety of the patient.

### [Disclosure]

### [Technical Problem]

Provided is an obstetric and gynecologic diagnosis apparatus including a chair part for obstetric and gynecologic diagnosis of which a shape automatically changes according to body information and obstetric and gynecologic information of a mother who is carrying a fetus.

Provided is an obstetric and gynecologic diagnosis method of diagnosing body information of a mother and a fetus by using the obstetric and gynecologic diagnosis apparatus.

### [Technical Solution]

Additional aspects will be set forth in part in the description which follows and, in part, will be apparent from the description, or may be learned by practice of the presented embodiments.

The invention is set out in the appended claims.

### [Description of Drawings]

These and/or other aspects will become apparent and more readily appreciated from the following description of the embodiments, taken in conjunction with the accompanying drawings in which:
FIG. 1 is a block diagram of a structure of an obstetric and gynecologic diagnosis apparatus according to an embodiment;
FIG. 2 is a perspective view of the obstetric and gynecologic diagnosis apparatus of FIG. 1;
FIG. 3A is a perspective view of the obstetric and gynecologic diagnosis apparatus for showing a vertical movement of a chair unit included in the obstetric and gynecologic diagnosis apparatus, according to an embodiment;
FIG. 3B is a perspective view of the obstetric and gynecologic diagnosis apparatus for showing rotational movements of an upper body support and a leg cradle included in the obstetric and gynecologic diagnosis apparatus, according to an embodiment;
FIG. 4A is a plan view of the obstetric and gynecologic diagnosis apparatus for showing planar movements of the ultrasound probe and the diagnosis part, according to an embodiment;
FIG. 4B is a side view of the obstetric and gynecologic diagnosis apparatus for showing vertical movements of the ultrasound probe and the diagnosis part, according to an embodiment;
FIG. 5A is a perspective view of the ultrasound probe and a probe holder according to an embodiment;
FIG. 5B is a block diagram showing some components of an obstetric and gynecologic diagnosis apparatus according to an embodiment;
FIG. 6 is a flowchart of an obstetric and gynecologic diagnosis method according to an embodiment;
FIGS. 7A to 7C are respectively a perspective view, a side view, and a plan view of the obstetric and gynecologic diagnosis apparatus in an initial state, according to an embodiment;
FIGS. 8A to 8C are respectively a perspective view, a side view, and a plan view of the obstetric and gynecologic diagnosis apparatus in a first diagnosis state, according to an embodiment;
FIGS. 9A and 9B are respectively a perspective view and a plan view of the obstetric and gynecologic diagnosis apparatus in a second diagnosis state, according to an embodiment;
FIGS. 10A to 10C are respectively a perspective view, a side view, and a plan view of the obstetric and gynecological diagnosis apparatus in a third diagnosis state, according to an embodiment;
FIG. 11 is a flowchart of an obstetric and gynecologic diagnosis method according to another embodiment; and
FIG. 12 is a flowchart of an obstetric and gynecologic diagnosis method according to another embodiment.

### [Mode for Invention]

The principle of the present disclosure is explained and embodiments are disclosed so that the scope of the present disclosure is clarified and one of ordinary skill in the art to which the present disclosure pertains may implement the present disclosure. The disclosed embodiments may have various forms.

Throughout the specification, like reference numerals or characters refer to like elements. In the specification, all elements of embodiments are not explained, but general matters in the technical field of the present disclosure or redundant matters between embodiments will not be described. Terms 'part' and 'portion' used herein may be implemented using software or hardware, and, according to embodiments, a plurality of 'parts' or 'portions' may be implemented using a single unit or element, or a single 'part' or 'portion' may be implemented using a plurality of units or elements. The operational principle of the present disclosure and embodiments thereof will now be described more fully with reference to the accompanying drawings.

Throughout the specification, the term 'object' is a thing to be imaged, and may include a patient, a mother, a fetus, or a part of a patient, mother, or fetus. For example, the object may include a part of a body organ of a mother, a fetus of the mother, a phantom, or the like.

Throughout the specification, a "user" may be, but is not limited to, a medical expert, such as a medical doctor, a nurse, a medical laboratory technologist, a medical image expert, or the like.

Throughout the specification, an "ultrasound image" refers to an image of an object processed based on ultrasound signals transmitted to the object and reflected therefrom.

Embodiments will now be described more fully hereinafter with reference to the accompanying drawings.

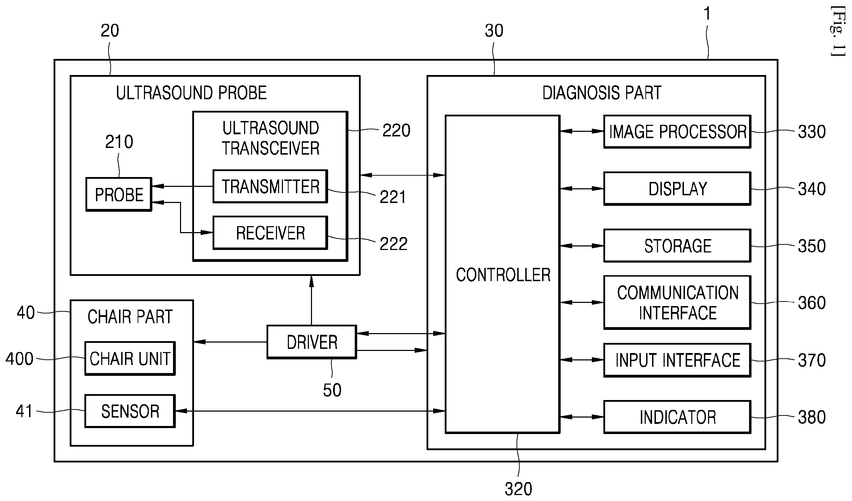

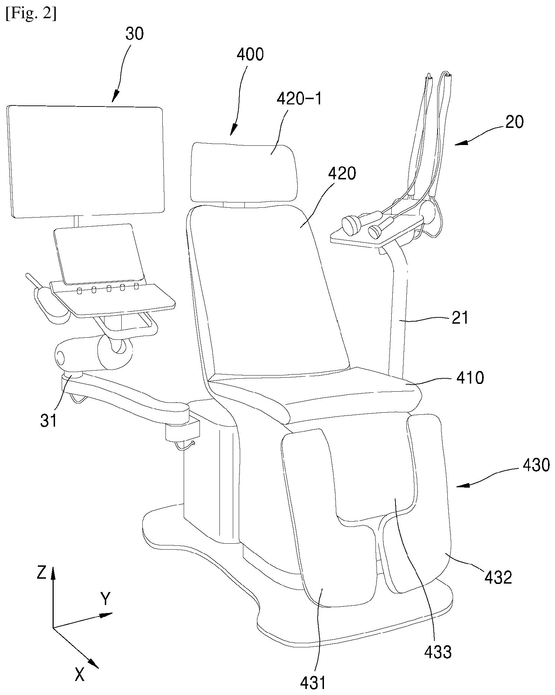

FIG. 1 is a block diagram of a structure of an obstetric and gynecologic diagnosis apparatus 1 according to an embodiment. FIG. 2 is a perspective view of the obstetric and gynecologic diagnosis apparatus 1.

Referring to FIGS. 1 and 2, the obstetric and gynecologic diagnosis apparatus 1 may include an ultrasound probe 20 including an ultrasound transceiver, and a diagnosis part 30 that may be used by a user to manipulate the obstetric and gynecologic diagnosis apparatus 1 to diagnose an object, and includes a chair part 40 on which the object is mountable.

According to an embodiment, the ultrasound probe 20 may include a probe 210 and an ultrasound transceiver 220 that transmits or receives ultrasound waves. The probe 210 may include a plurality of transducers. The plurality of transducers may transmit ultrasound signals to an object according to a transmission signal applied by a transmitter 221 included in the ultrasound transceiver 220. The plurality of transducers may receive ultrasound signals reflected by the object and may form a reception signal. The probe 210 may be integrated with the obstetric and gynecologic diagnosis apparatus 1, or may be separate from the obstetric and gynecologic diagnosis apparatus 1 and may be connected thereto via wired and/or wireless communication. In addition, the obstetric and gynecologic diagnosis apparatus 1 may include one probe 210 or a plurality of probes 210 according to embodiments.

According to an embodiment, the diagnosis part 30 may include a controller 320 for controlling the obstetric and gynecologic diagnosis apparatus 1, an image processor 330 for processing a received signal into an image, a display 340 for outputting the image, a storage 350, a communication interface 360, and an input interface 370.

The controller 320 may control all operations of the obstetric and gynecologic diagnosis apparatus 1 and signal transfer among the internal components of the obstetric and gynecologic diagnosis apparatus 1. The controller 320 may include a memory configured to store programs or data for performing functions of the obstetric and gynecologic diagnosis apparatus 1, and a processor configured to process the programs or the data. The controller 320 may receive a control signal from the input interface 370 or an external device to control an operation of the obstetric and gynecologic diagnosis apparatus 1.

The signal processor 330 generates an ultrasound image by using ultrasound data generated by a receiver 222 included in the ultrasound transceiver 220.

The display 340 may display the generated ultrasound image and various pieces of information processed in the obstetric and gynecologic diagnosis apparatus 1. The display 340 may include one display or a plurality of displays according to embodiments, for example, a first display for the user and a second display for the object. In this case, the display may be coupled with a touch panel and thus may be implemented as a touch screen.

The storage 350 may store, for example, various pieces of data or various programs for driving and controlling the obstetric and gynecologic diagnosis apparatus 1, input/output ultrasound data, obtained ultrasound images, identification (ID) information and body information of the object, and ID information and body information of the user.

Herein, the ID information of the object and the user means at least one of all types of information capable of identifying the object and the user, for example, a name, a resident registration number, a birth date, a personal ID number, a personal ID code, and biometric information (e.g., a face, an iris, and a fingerprint). Herein, the body information of the object means all pieces of body information of the object necessary for an obstetric and gynecologic treatment, including a gestation period of a pregnant woman, and optionally the number of fetuses carried by the pregnant woman, the location of a fetus, the weight of the pregnant woman, the height of the pregnant woman, the body temperature of the pregnant woman, the examination history of the pregnant woman, and the medical history of the pregnant woman. Herein, the body information of the user means all pieces of body information of the user necessary during a diagnosing process, for example, a surgical procedure posture of a surgical operator, the height and sitting height thereof, the arm length thereof, and an eye position thereof.

The obstetric and gynecologic diagnosis apparatus 1 may include the communication interface 360 and may be connected to external devices, such as, a central server, a medical apparatus, and a portable device (smartphone, tablet personal computer (PC), wearable device, etc.), via the communication interface 360.

The communication interface 360 may include one or more components that enable communication with an external device. For example, the communication interface 360 may include at least one of a short distance communication module, a wired communication module, and a wireless communication module.

For example, the communication interface 360 may transmit the ID information of the object and the user to an external device, for example, a central server, and the external device transmits data related to body information of the object and the user according to the received ID information of the object and the user to the controller 320 so that the controller 320 controls the obstetric and gynecologic diagnosis apparatus 1 according to the received data related to the body information of the object and the user. The external device may include a recording medium having recorded thereon the data related to the body information of the object and the user.

The input interface 370 may receive a user input for controlling the obstetric and gynecologic diagnosis apparatus 1. For example, the user may input, for example, the ID information of the object, the ID information of the user, or a manipulation signal (which will be described later below) for adjusting a position of the chair part 40, to the input interface 370. The user input may include, but is not limited to, an input for manipulating a button, a key pad, a mouse, a trackball, a jog switch, a knob, or the like, an input for touching a touch pad or a touch screen, a voice input, a motion input, and a biometric data input.

An indicator 380 is a display capable of displaying an operating state of the obstetric and gynecologic diagnosis apparatus 1 to the object. For example, the indicator 380 may display various operating states of the obstetric and gynecologic diagnosis apparatus 1 by using a change in the color of a visual indicator, for example, a light-emitter, as shown in FIG. 2, or may display the various operating states of the obstetric and gynecologic diagnosis apparatus 1 by using an auditory indicator. However, embodiments of the present disclosure are not limited thereto, and the indicator 380 may be implemented by using the other display methods, such as a character and an image.

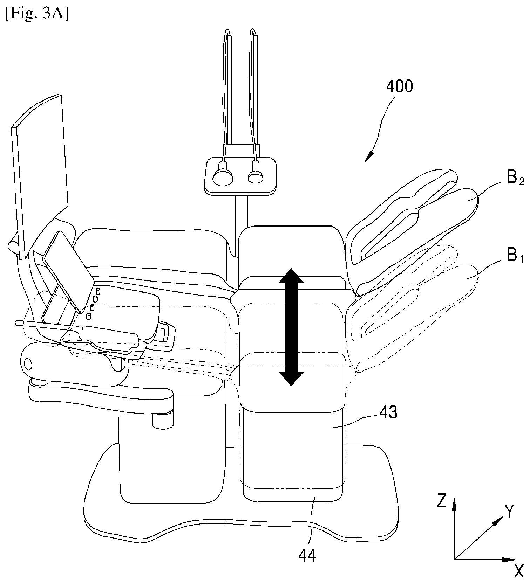

The chair part 40 includes a chair unit 400 on which the object is mountable, and at least one sensor 41 capable of obtaining body information of the object. The chair unit 400 is thick enough that the object can be seated thereon. A length direction of the chair unit 400 is parallel to a length or height direction of the object. The chair unit 400 may be fixed to the floor or may be movable on the floor. For example, the chair unit 400 is vertically movable to ascend or descend the object, and may be inclined to adjust a diagnosis angle with respect to the object. The movement of the chair unit 400 to diagnose the object will be described later in more detail with reference to FIG. 3A.

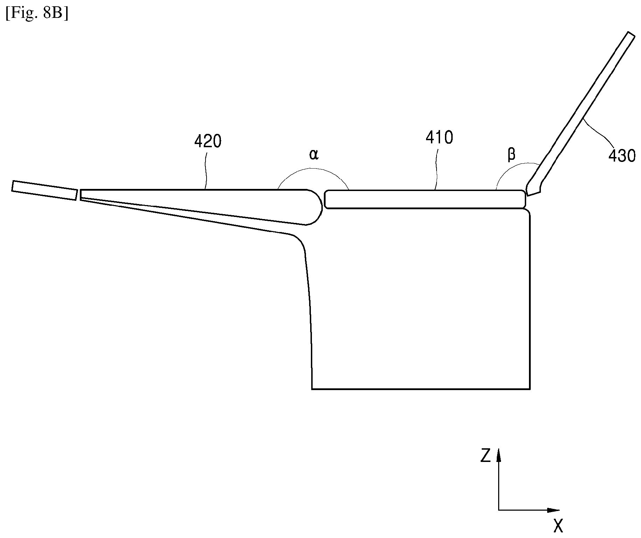

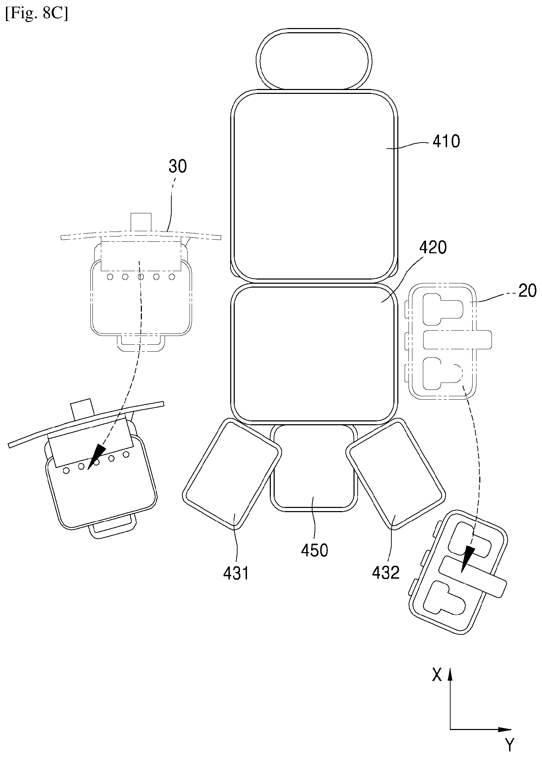

The chair unit 400 includes a seat 410, and an upper body support 420 of which an inclination is adjustable with respect to the seat 410. The seat 410 supports a lower body of the object, and the upper body support 420 supports an upper body of the object. The upper body support 420 may include a head support 420-1 for supporting the head of the object. The head support 420-1 is fixed to the upper body support 420 to be detachable therefrom, and may be separated from the upper body support 420 according to a diagnosis state. The chair unit 400 also includes a leg cradle 430 for supporting the legs of the object. According to an embodiment, the leg cradle 430 may include a first cradle 431 and a second cradle 432 capable of respectively supporting both legs of the object, namely, the right leg and the left leg. A cover support 433, which is detachable, may be arranged between the first cradle 431 and the second cradle 432. A secretion container 450 of FIG. 8C for temporarily storing secretions of the object produced while the object is being diagnosed may be arranged below the cover support 433. The cover support 433 may be fixed between the first cradle 431 and the second cradle 432 to support the object or may be separated from the chair unit 400 to expose the secretion container 450, according to diagnosis states of the object.

For example, the user may adjust the angle of the upper body support 420 with respect to the seat 410 or adjust the angle of the leg cradle 430 with respect to the seat 410, according to diagnosis states of the object. The first cradle 431 and the second cradle 432 included in the leg cradle 430 may be adjusted at various angles to be spaced apart from each other according to a diagnosis state of the object.

The sensor 41 is a sensing device capable of sensing the state of the object mounted on the chair unit 400. For example, the sensor 41 may include a weight sensor capable of measuring measured information of the object, for example, the weight of the object, in real time, a temperature sensor capable of sensing a temperature change of the object, a time sensor capable of sensing and calculating a seating duration time, or an operation sensor capable of sensing a sudden seating state change of the object. Accordingly, the sensor 41 may sense measured information of the object and a sudden seating state change of the object occurring during a diagnosis of the object, and may transmit the measured information of the object and location movement information of the object to the controller 320.

Referring back to FIG. 1, the driver 50 may generate a driving force capable of moving the ultrasound probe 20, the diagnosis part 30, and the chair part 40 according to the control signal of the controller 320. When the object is a mother who is carrying a fetus, her appearance rapidly changes according to growth of the fetus, and diagnostic items also change according to the growth of the fetus. According to a change in a diagnostic posture of the mother due to changes in the external appearance of the mother and the diagnostic items, the shape and location of the chair part 40, on which the mother is seated to receive an obstetric and gynecologic diagnosis, need to be changed, and those of the ultrasound probe 20 and the diagnosis part 30 also need to be changed in order to provide usage convenience to the user. The formation of the control signal capable of controlling the driver 50 according to the body information of the object and the body information of the user, and the automatic movement of the ultrasound probe 20, the diagnosis part 30, and the chair part 40 in accordance with the diagnosis state of the object will now be described in more detail.

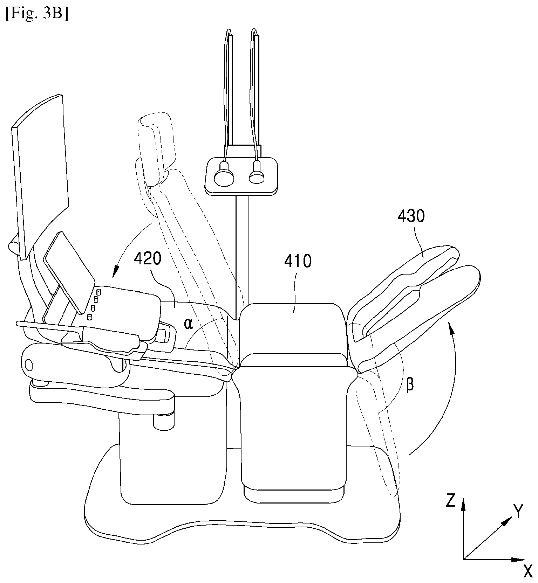

FIG. 3A is a perspective view of the obstetric and gynecologic diagnosis apparatus 1 for showing a vertical movement of the chair unit 400. FIG. 3B is a perspective view of the obstetric and gynecologic diagnosis apparatus 1 for showing rotational movements of the upper body support 420 and the leg cradle 430.

According to an embodiment, the user may perform an obstetric and gynecologic diagnosis using the obstetric and gynecologic diagnosis apparatus 1 while standing or sitting according to diagnosis states of the object. In addition, the height of an object and a ratio between the upper body and the lower body of the object may differ according to individuals. Thus, according to obstetric and gynecologic diagnosis states of the object and the body information of the object and the user, the object that is being diagnosed may be mounted on the chair unit 400 to be at different locations from the ground or to maintain various postures.

Referring to FIG. 3A, the chair unit 400 may move vertically with respect to the ground. The ground refers to a support surface on which a bottom of the obstetric and gynecologic diagnosis apparatus 1 is supported, for example, the floor of a building on which the obstetric and gynecologic diagnosis apparatus 1 is supported or the floor of a diagnosis room where an obstetric and gynecologic diagnosis is performed.

For example, when the chair unit 400 is arranged at a first location B1 that is relatively low from the ground, a slide part 43 fixed to the bottom of the chair unit 400 may be moved downwards along a Z-axis by a slide guide part 44 fixed to the ground. At this time, the driver 50 of FIG. 1 may generate a driving force according to the control signal received from the controller 320 and automatically move the chair unit 400. Accordingly, the object mounted on the chair unit 400 may also be moved downwards along the Z-axis and be positioned at a low location from the ground.

On the other hand, when the chair unit 400 is arranged at a second location B2 that is relatively high from the ground, the slide part 43 fixed to the bottom of the chair unit 400 may be moved upwards along the Z-axis by the slide guide part 44 fixed to the ground. At this time, the driver 50 of FIG. 1 may generate a driving force according to the control signal received from the controller 320 and automatically move the chair unit 400. Accordingly, the object mounted on the chair unit 400 may also be moved upwards along the Z-axis and be positioned at a high location from the ground.

Referring to FIG. 3B, the upper body support 420 and the leg cradle 430 of the chair unit 400 may rotate with respect to the seat 410 such that the object may maintain various postures.

For example, the upper body support 420 may rotate about an axis Y with respect to the seat 410. For example, the upper body support 420 may be rotated to have a first angle α that is no less than 90° and no more than 180°, with respect to the seat 410. At this time, the driver 50 of FIG. 1 may generate a driving force according to the control signal received from the controller 320 and transmit the driving force to a driving force transmitter (not shown) arranged on the bottom of the chair unit 410 to thereby automatically rotate the upper body support 420. Accordingly, the upper body of the object mounted on the chair unit 400 may also be rotated with respect to the lower body of the object supported by the chair unit 410 and thus be positioned at a diagnosis location where the object sits or lies.

The leg cradle 430 may rotate about the axis Y with respect to the seat 410. For example, the leg cradle 430 may be rotated to have a second angle β that is no less than 100° and no more than 270° with respect to the seat 410. At this time, the driver 50 of FIG. 1 may generate a driving force according to the control signal received from the controller 320 and transmit the driving force to the driving force transmitter arranged on the bottom of the chair unit 410 to thereby automatically rotate the leg cradle 430. Accordingly, the legs of the object mounted on the chair unit 400 may be rotated to be positioned at a certain diagnosis location.

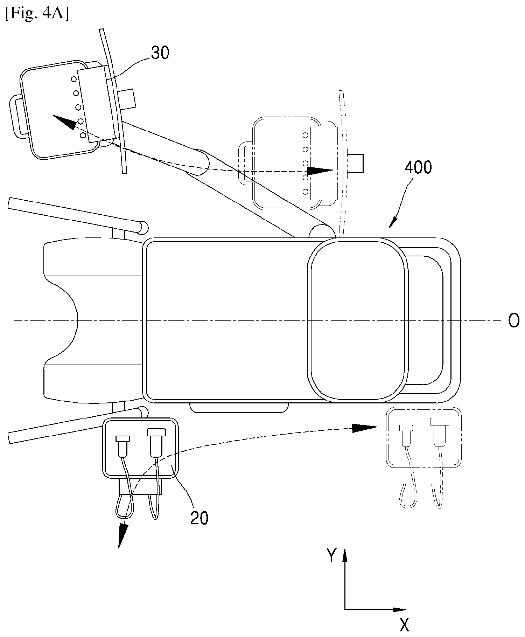

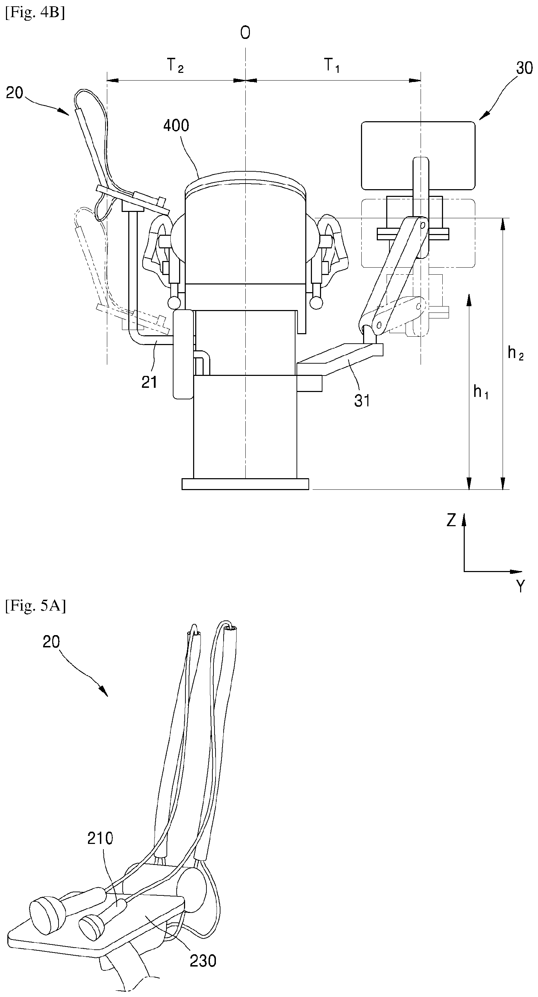

FIG. 4A is a plan view of the obstetric and gynecologic diagnosis apparatus 1 for showing planar movements of the ultrasound probe 20 and the diagnosis part 30. FIG. 4B is a side view of the obstetric and gynecologic diagnosis apparatus 1 for showing vertical movements of the ultrasound probe 20 and the diagnosis part 30.

According to an embodiment, the user may perform an obstetric and gynecologic diagnosis using the obstetric and gynecologic diagnosis apparatus 1 by being located adjacent to the upper body or the lower body of the object according to diagnosis states. Accordingly, to improve the usage convenience of the user, the locations of the ultrasound probe 20 and the diagnosis part 30 used by the user may vary according to obstetric and gynecologic diagnosis states of the object and the body information of the user.

Referring to FIGS. 4A and 4B, the ultrasound probe 20 and the diagnosis part 30 may be arranged on opposite sides of the chair unit 400. In this case, the ultrasound probe 20 and the diagnosis part 30 may be connected to each other to be movable relative to the chair unit 400. For example, the ultrasound probe 20 and the diagnosis part 30 arranged on opposite sides of the chair unit 400 may be spaced apart from a center line of the chair unit 400 parallel to a length direction thereof by distances T2 and T1, respectively, for example, by distances that are no less than 30 cm and no more than 70 cm, according to a diagnosis state of the object. In this case, the ultrasound probe 20 and the diagnosis part 30 may move from above the upper body support 420 to below the leg cradle 430 in an X-axis direction. The ultrasound probe 20 and the diagnosis part 30 may be arranged to be spaced apart from the ground by heights h1 and h2, for example, heights that are no less than 70 cm and no more than 120 cm, according to diagnosis states of the object. At this time, the driver 50 of FIG. 1 may generate a driving force according to the control signal received from the controller 320 and transmit the driving force to a first connector 21 and a second connector 31 each implemented as a plurality of link parts, to thereby automatically move the ultrasound probe 20 and the diagnosis part 30. Accordingly, the ultrasound probe 20 and the diagnosis part 30 may be positioned at diagnosis locations corresponding to each diagnosis state.

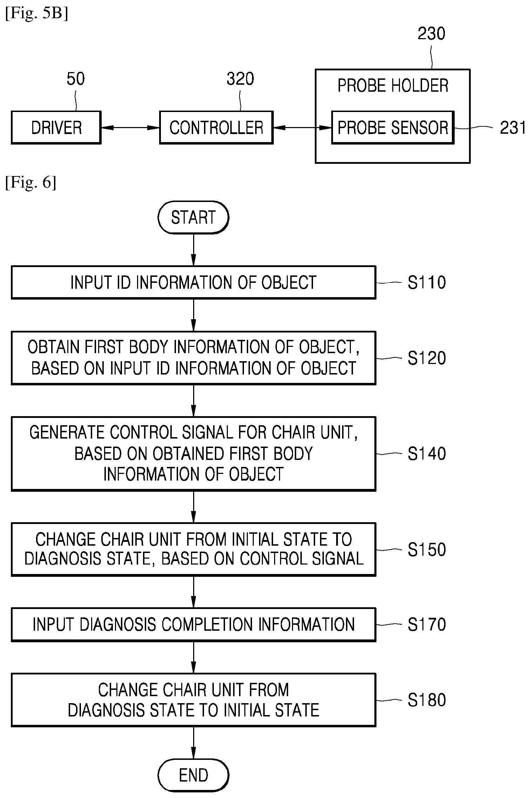

FIG. 5A is a perspective view of the ultrasound probe 20 and a probe holder 230 according to an embodiment. FIG. 5B is a block diagram showing some components of an obstetric and gynecologic diagnosis apparatus according to an embodiment.

Referring to FIGS. 5A and 5B, the ultrasound probe 20 may further include the probe holder 230 capable of holding the probe 210, in addition to the probe 210 and the ultrasound transceiver 220. The user may use the probe 210 to diagnose the object, and may hold the probe 210 on the probe holder 230 after the diagnosis is interrupted or completed.

The probe holder 230 may include a probe sensor 231 that detects whether the probe 210 has been held on the probe holder 230. The probe sensor 231 may be arranged on at least one of a plurality of internal walls of the probe cradle 230, but the arrangement of the probe sensor 231 is not limited thereto. For example, the probe sensor 231 may be a weight sensor that senses the weight of the probe 210, a piezoelectric sensor or micro-switch pressed by the probe 210, or an optical sensor. For example, when the probe sensor 231 senses that the probe 210 is held on the probe holder 230 for a certain period of time, for example, 60 seconds or more, it may be recognized that the diagnosis of the object has been completed.

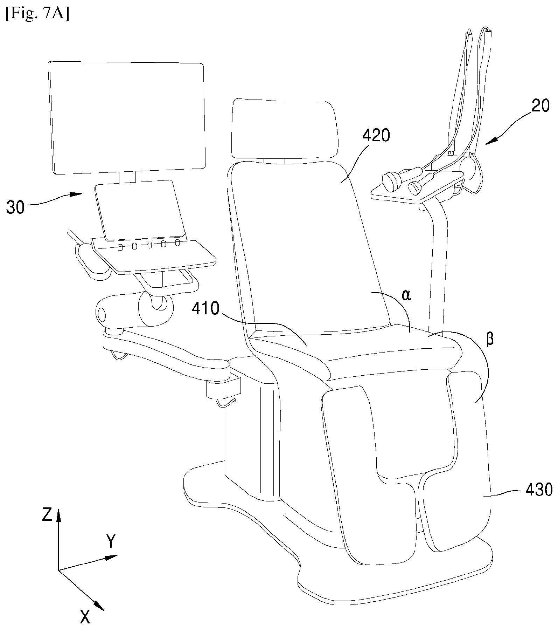

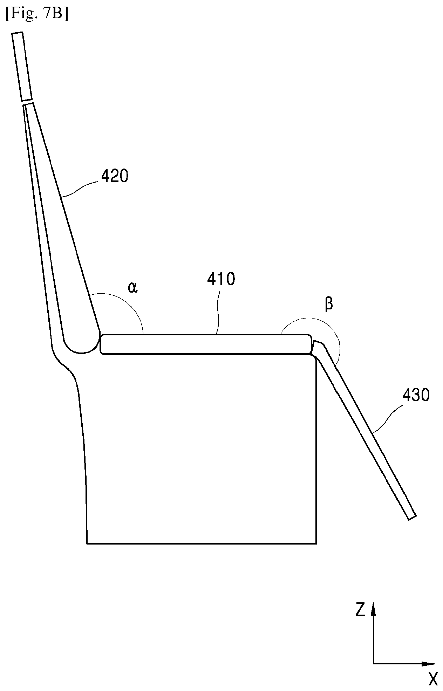

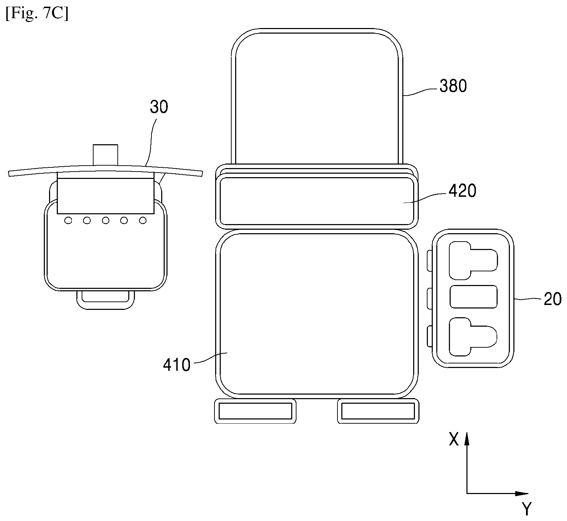

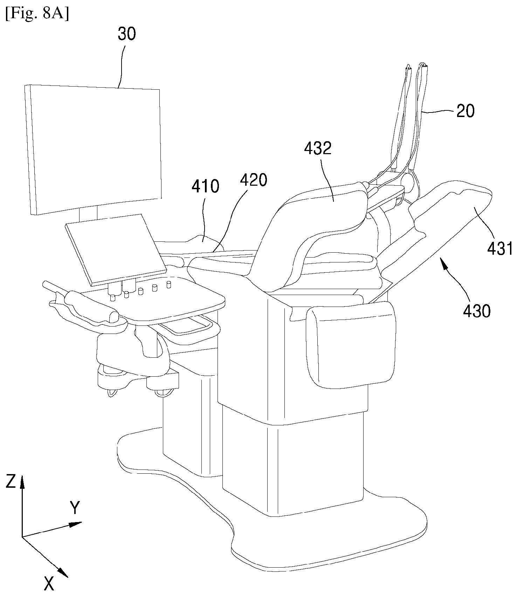

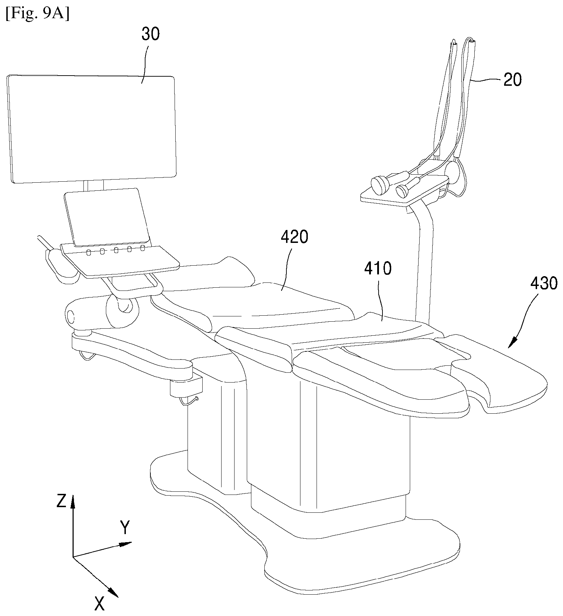

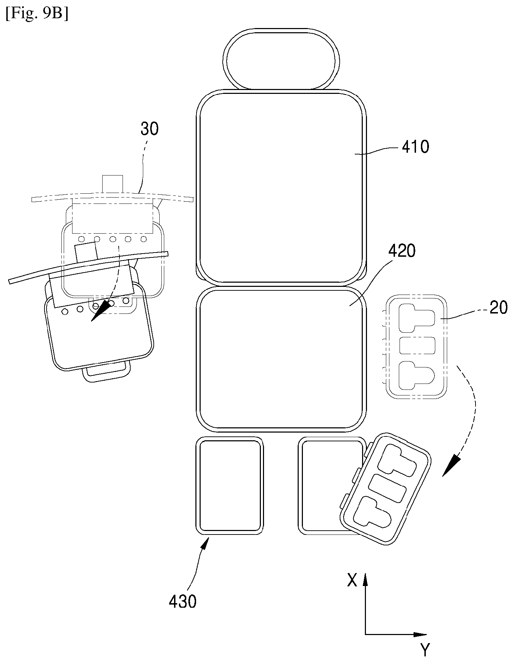

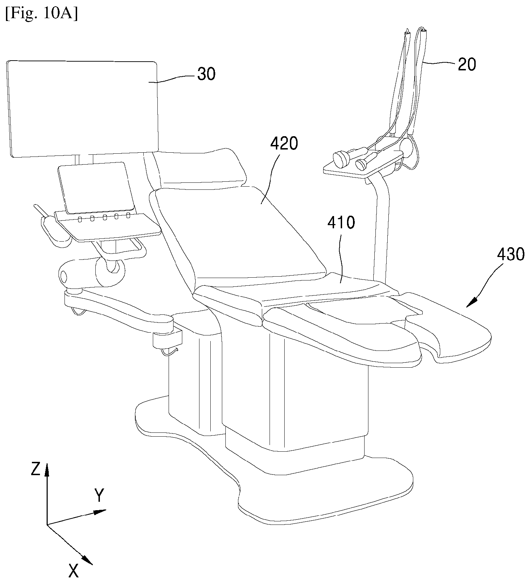

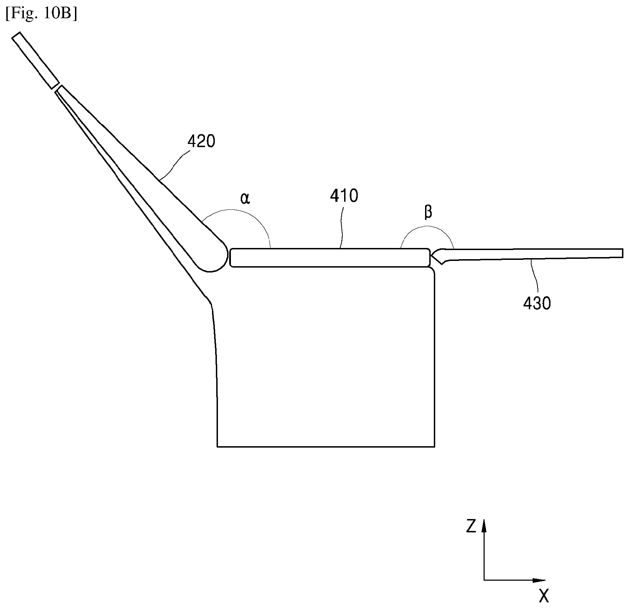

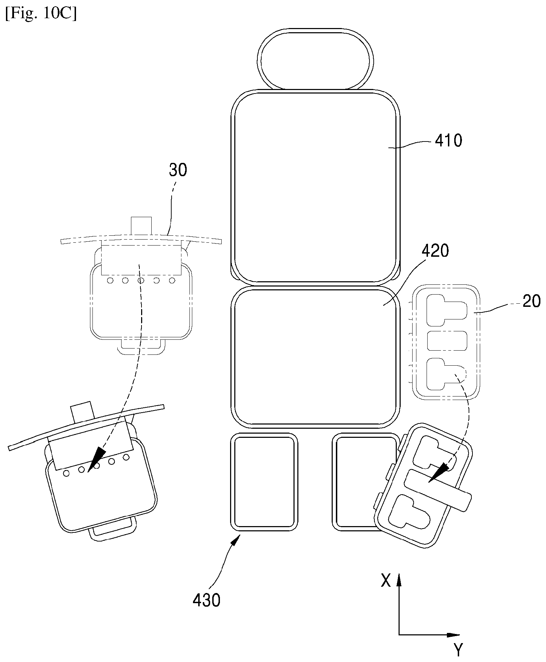

FIG. 6 is a flowchart of an obstetric and gynecologic diagnosis method according to an embodiment. FIGS. 7A to 7C are respectively a perspective view, a side view, and a plan view of the obstetric and gynecologic diagnosis apparatus 1 in an initial state, according to an embodiment. FIGS. 8A to 8C are respectively a perspective view, a side view, and a plan view of the obstetric and gynecologic diagnosis apparatus 1 in a first diagnosis state, according to an embodiment. FIGS. 9A and 9B are respectively a perspective view and a plan view of the obstetric and gynecologic diagnosis apparatus 1 in a second diagnosis state, according to an embodiment. FIGS. 10A to 10C are respectively a perspective view, a side view, and a plan view of the obstetric and gynecologic diagnosis apparatus 1 in a third diagnosis state, according to an embodiment.

Referring to FIG. 6, in operation S110, the ID information of the object is input via the input interface 370 included in the diagnosis part 30. For example, the ID information of the object means, as described above, at least one of all types of information capable of identifying the object, for example, a name, a resident registration number, a birth date, a personal ID number, a personal ID code, and biometric information (e.g., a face, an iris, and a fingerprint).

In operation S120, first body information of the object is obtained based on the ID information of the object input via the input interface 370. According to an embodiment, the controller 320 may obtain first body information of the object corresponding to ID information of the object stored in the storage 350 or an external device, by using the ID information of the object input via the input interface 370. At this time, the storage 350 included in the diagnosis part 30 or an external device communicable with the diagnosis part 30 via the communication interface 360 may store the first body information of the object. The first body information of the object means all pieces of pre-storable body information of the object necessary for an obstetric and gynecologic treatment, including a gestation period of a mother who is carrying a fetus, and optinally the number of fetuses, the location of a fetus, the weight of the mother, the height of the mother, the body temperature of the mother, the examination history of the mother, and the medical history of the mother.

In operation S140, a control signal for the chair unit 400 is generated based on the obtained first body information of the object. According to an embodiment, the controller 320 may ascertain a diagnosis state and body information of the object by using the obtained first body information of the object. At this time, the chair unit 400 may be changed according to the diagnosis state and the body information of the object, and the controller 320 may generate a control signal capable of generating a driving force for transforming the chair unit 400.

In operation S150, based on the control signal generated by the controller 320, the chair unit 400 is changed from an initial state to a diagnosis state. According to an embodiment, based on the control signal generated by the controller 320, the driver 50 may generate a driving force and change the chair unit 400 from the initial state to the diagnosis state. For example, the chair unit 400 may be changed from the initial state to the first through third diagnosis states, according to the body information of the object, including a gestation period. At this time, a visual indicator or an auditory indicator as the indicator 380 may make the object know that the change of the chair unit 400 from an initial state to a diagnosis state.

According to an embodiment, in the initial state, the chair unit 400 may be a structure in which it is easy for the object to sit on the chair unit 400, as shown in FIGS. 7A to 7C. For example, in the initial state, the upper body support 420 may be arranged to have a first angle α of 90° to 120° counterclockwise with respect to the seat 410. At this time, the leg cradle 430 may be arranged to have a second angle β of 240° to 360° clockwise with respect to the seat 410. The ultrasound probe 20 and the diagnosis part 30 may be arranged adjacent to the seat 410 such that the object is not interfered while sitting down on the chair unit 400.

According to an embodiment, when the object, namely, a pregnant woman, is in an early stage (10 weeks or less of pregnancy) as shown in FIGS. 8A to 8C, the first diagnosis state may be a structure of the chair unit 400 for diagnosing a lower body part of the object. For example, in the first diagnosis state, the upper body support 420 may be inclined to have a flat state, namely, a first angle α of 180°, with respect to the seat 410. However, embodiments of the present disclosure are not limited thereto, and, as necessary, the upper body support 420 may be inclined at a first angle α, for example, an angle of 110° to 180°, with respect to the seat 410, during a diagnosis. The leg cradle 430 may also be inclined to have a second angle β of 120° to 180° clockwise with respect to the seat 410. The seat 410 may also be inclined to have an angle of 0° to 20° with respect to the ground. At this time, the first cradle 431 and the second cradle 432 included in the leg cradle 430 may be moved such that one of the first cradle 431 is spaced apart from one end of the second cradle 432, and the secretion container 450 may be arranged between the first cradle 431 and the second cradle 432.

At this time, the chair unit 400 may be arranged to ascend or descend from the ground according to the height of the user and a diagnosis type. For example, the ultrasound probe 20 and the diagnosis part 30 may also be arranged to ascend or descend from the ground according to the height of the user and the diagnosis type, and may be arranged more adjacent to the leg cradle 430 than to the seat 410 considering the arm length of the user and the diagnosis type, thereby maximizing the usage convenience of the user.

According to an embodiment, when the object, namely, a pregnant woman, is in a middle stage (10 to 30 weeks of pregnancy) as shown in FIGS. 9A and 9B, the second diagnosis state may be in a structure of the chair unit 400 for diagnosing an abdomen part of the object. For example, in the second diagnosis state, the seat 410, the upper body support 420, and the leg cradle 430 may be arranged on a plane. In other words, the upper body support 420 may be inclined to have a flat state, namely, a first angle α of 180°, with respect to the seat 410. However, embodiments of the present disclosure are not limited thereto, and, as necessary, the upper body support 420 may be inclined at a first angle α, for example, an angle of 110° to 180°, with respect to the seat 410, during a diagnosis. Mattes related to the ascending and descending of the chair unit 400 and the movements of the ultrasound probe 20 and the diagnosis part 30 according to the height of the user and the diagnosis type are substantially the same as those in the first diagnosis state, and thus descriptions thereof will be omitted.

According to an embodiment, when the object, namely, a pregnant woman, is in a late stage (30 weeks or more of pregnancy) as shown in FIGS. 10A to 10C, the third diagnosis state may be a structure of the chair unit 400 for diagnosing an abdomen part of the object. When the state of a pregnant woman is in the later stages of pregnancy, it is hard for the pregnant woman to lie flat on her back due to the weight of the fetus. Accordingly, considering the diagnosis convenience of the object, the upper body support 420 needs to move relative to the seat 410. For example, in the third diagnosis state, the upper body support 420 may be inclined to have a first angle α of 110° to 170° counterclockwise with respect to the seat 410. The leg cradle 430 may be arranged on the same plane as the seat 410. Mattes related to the ascending and descending of the chair unit 400 and the movements of the ultrasound probe 20 and the diagnosis part 30 according to the height of the user and the diagnosis type are substantially the same as those in the first diagnosis state, and thus descriptions thereof will be omitted.

Referring back to FIG. 6, in operation S170, when the diagnosis of the object by the user is completed, diagnosis completion information is input. According to an embodiment, when the diagnosis of the object by the user is completed, the user may hold the probe 210 on the probe holder 230 as shown in FIG. 5A. When the probe sensor 231 senses that the probe 210 is held on the probe holder 230 for a certain period of time, it may be recognized that the diagnosis of the object has been completed, and, diagnosis completion information indicating that the diagnosis of the object has been completed may be input. However, embodiments of the present disclosure are not limited thereto, and the diagnosis completion information may be directly input by the user by using the input interface 370.

In operation S180, when the diagnosis completion information is input, the chair unit 400 is transformed from the diagnosis state to the initial state, based on the control signal generated by the controller 320. According to an embodiment, based on the control signal generated by the controller 320, the driver 50 may generate a driving force and transform the chair unit 400 from the diagnosis state to the initial state. At this time, a visual indicator or an auditory indicator as the indicator 380 may make the object know the completion of the diagnosis of the object and the transformation of the chair unit 400 from the diagnosis state to the initial state.

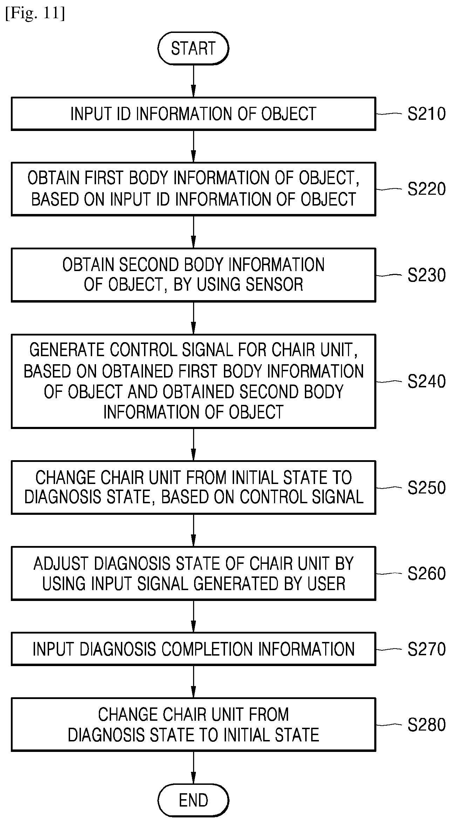

FIG. 11 is a flowchart of an obstetric and gynecologic diagnosis method according to another embodiment. Operations other than operations S230, S240, and S260 of the obstetric and gynecologic diagnosis method of FIG. 11 are substantially the same as those of FIG. 6. Thus, for convenience of explanation, operations S230, S240, and S260 of FIG. 11 different from FIG. 6 will be focused.

Referring to FIG. 11, in operation S230, second body information of the object is obtained by the sensor 41. The sensor 41 senses body information of the object, including at least one of a current weight, a body temperature, or a sudden seating state change of the object occurring during a diagnosis of the object, from the object seated on the chair unit 400, and may transmit measured body information of the object and location movement information of the object to the controller 320. When the object is a mother who is carrying a fetus, a sharp weight change and a sharp body temperature change may occur according to a growth of the fetus. A sudden seating state change of the object may also occur during a diagnosis of the object. The second body information of the object is obtained by sensing such a real-time change and may be different from the first body information of the object pre-stored in the storage 350.

In operation S240, a control signal for the chair unit 400 is generated based on the obtained first and second body information of the object. According to an embodiment, the controller 320 may ascertain a diagnosis state and body information of the object by using the first body information of the object obtained using information stored in the storage 350 and the second body information of the object obtained in real time by using the sensor 41. At this time, the chair unit 400 may be changed according to the diagnosis state and the body information of the object, and the controller 320 may generate a control signal capable of generating a driving force for transforming the chair unit 400.

In operation S260, the diagnosis state of the chair unit 400 is adjusted using an input signal generated by the user. According to an embodiment, as described above, based on the control signal generated by the controller 320, the driver 50 may generate a driving force and change the chair unit 400 from the initial state to the diagnosis state. However, the change of the chair unit 400 to the diagnosis state based on the first body information and the second body information of the object may not completely coincide with a diagnosis environment. At this time, the user may input to the input interface 370 an input signal for transforming the chair unit 400. The input signal may be transmitted to the controller 320, and, based on the control signal generated by the controller 320,6 the driver 50 may generate a driving force and adjust the diagnosis state of the chair unit 400 such that the diagnosis state of the chair unit 400 coincides with a current diagnosis environment.

As described above, the diagnosis state of the chair unit 400 may be adjusted in three stages by using the first body information of the object pre-stored in the storage 350, using the second body information of the object obtained in real time by the sensor 41, and using the input signal generated by the user, and thus diagnosis convenience of the object may be secured. However, embodiments of the present disclosure are not limited thereto, and the adjustment of the diagnosis state of the chair unit 400 in a stage of using the second body information of the object or using the input signal generated by the user from among the three stages may be omitted according to a diagnosis environment and an adjustment necessity.

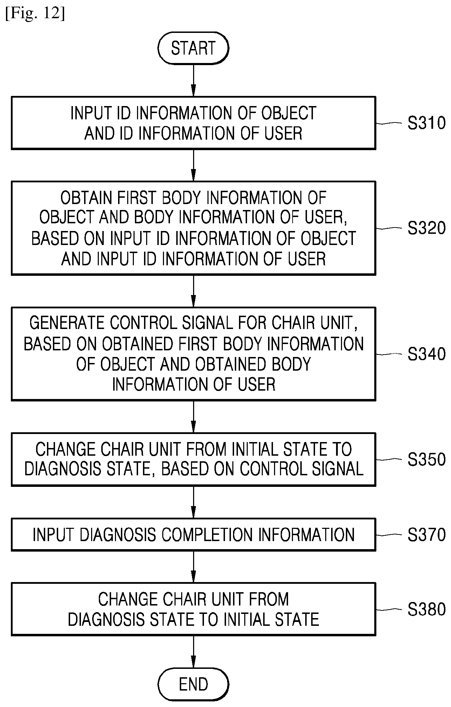

FIG. 12 is a flowchart of an obstetric and gynecologic diagnosis method according to another embodiment. Operations other than operations S310, S320, and S340 of the obstetric and gynecologic diagnosis method of FIG. 12 are substantially the same as those of FIG. 6. Thus, for convenience of explanation, operations S310, S320, and S340 of FIG. 12 different from FIG. 6 will be focused.

Referring to FIG. 12, in operation S310, the ID information of the object and the ID information of the user are input via the input interface 370 included in the diagnosis part 30. For example, the ID information of the object and the ID information of the user means at least one of all types of information capable of identifying the object and the user, for example, a name, a resident registration number, a birth date, a personal ID number, a personal ID code, and biometric information (e.g., a face, an iris, and a fingerprint).

In operation S320, first body information of the object and body information of the user are obtained based on the ID information of the object and the ID information of the user input via the input interface 370. According to an embodiment, the controller 320 may obtain first body information of the object corresponding to ID information of the object stored in the storage 350 or an external device and body information of the user corresponding to the ID information of the user, by using the ID information of the object and the ID information of the user input via the input interface 370. At this time, the storage 350 included in the diagnosis part 30 or an external device communicable with the diagnosis part 30 via the communication interface 360 may store the first body information of the object and the body information of the user. The first body information of the object means all pieces of pre-storable body information of the object necessary for an obstetric and gynecologic treatment, including a gestation period of a mother who is carrying a fetus, the number of fetuses, the location of a fetus, the weight of the mother, the height of the mother, the body temperature of the mother, the examination history of the mother, and the medical history of the mother. The body information of the user means all pieces of body information of the user necessary to perform an obstetric and gynecologic treatment by manipulating the probe 210, for example, a surgical procedure posture of the user, the arm length of a surgical operator, an eye position of the surgical operator, the height thereof, the sitting height thereof, and the length of the arms thereof.

In operation S340, a control signal for the chair unit 400 is generated based on the obtained first body information of the object and the body information of the user. According to an embodiment, the controller 320 may ascertain a diagnosis state and body information of the object and the body information of the user by using the first body information of the object obtained using information stored in the storage 350 and the ID information of the user. At this time, the chair unit 400 may be changed according to the diagnosis state and the body information of the object and the body information of the user, and the controller 320 may generate a control signal capable of generating a driving force for transforming the chair unit 400.

In operation S350, based on the control signal generated by the controller 320, the chair unit 400 is changed from an initial state to a diagnosis state. According to an embodiment, based on the control signal generated by the controller 320, the driver 50 may generate a driving force and change the chair unit 400 from the initial state to the diagnosis state. For example, the chair unit 400 may be changed from the initial state to the first through third diagnosis states, according to the body information of the object, including a gestation period. At this time, a visual indicator or an auditory indicator as the indicator 380 may make the object know that the change of the chair unit 400 from an initial state to a diagnosis state. At this time, the chair unit 400 may be arranged to ascend or descend from the ground according to the body information of the user, for example, the height and sitting height of the user, as shown in FIGS. 8A to 10B. For example, the ultrasound probe 20 and the diagnosis part 30 may also be arranged to ascend or descend from the ground according to the height, sitting height, and arm length of the user, a diagnosis type, and a diagnosis posture, and may be arranged more adjacent to the leg cradle 430 than to the seat 410 considering the arm length of the user and the diagnosis type, thereby maximizing the usage convenience of the user.

An obstetric and gynecologic diagnosis apparatus according to an embodiment is automatically transformed according to body information of a pregnant woman and obstetric and gynecologic information thereof, thereby improving convenience of the pregnant woman or the user and minimizing a diagnosis period of time and shame and anxiety of the pregnant woman.

While an obstetric and gynecologic diagnosis apparatus and an obstetric and gynecologic diagnosis method using the same have been particularly shown and described with reference to exemplary embodiments thereof, it will be understood by those of ordinary skill in the art that various changes in form and details may be made therein without departing from the scope as defined by the following claims.

## Claims

1. An obstetric and gynecologic diagnosis apparatus (1) comprising:
a chair unit (400) on which an object is to be mounted, the chair unit (400) comprising an upper body support (420), a seat (410), and a leg cradle (430) sequentially arranged in one direction and connected to each other;
a controller (320) configured to generate a control signal for moving at least one of the upper body support (420), the seat (410), and the leg cradle (430) according to first body information of the object including a gestation period of the object;
a driver (50) configured to generate a driving force for moving at least one of the upper body support (420), the seat (410), and the leg cradle (430) according to the control signal; and
at least one sensor (41) configured to sense second body information of the object, the second body information being at least one of a weight, a body temperature, a sitting state, and a sitting duration time;
wherein a relative position of the upper body support (420), the seat (410), and the leg cradle (430) is changed according to a stage of the gestation period of the object included in the first body information,
wherein the controller (320) generates the control signal for moving at least one of the upper body support (420), the seat (410), and the leg cradle (430) according to the first body information of the object and the second body information of the object.

2. The obstetric and gynecologic diagnosis apparatus (1) of claim 1, further comprising:
a storage (350) configured to store the first body information of the object; and
an input interface (370) configured to input identification (ID) information of the object,
wherein the first body information of the object is identified by the ID information of the object, and the ID information of the object is at least one of name information of the object, fingerprint information of the object, face information of the object, and an ID code corresponding to the object.

3. The obstetric and gynecologic diagnosis apparatus (1) of claim 2, wherein user ID information comprising information of a user is input to the input interface (370), and the controller (320) generates a control signal for moving at least one of the upper body support (420), the seat (410), and the leg cradle (430) according to the first body information of the object and the information of the user.

4. The obstetric and gynecologic diagnosis apparatus (1) of claim 1, wherein the chair unit (400) is arranged to be vertically movable with respect to the ground, the upper body support (420) is connected to the seat (410) to be rotatable about one axis, and the leg cradle (430) is connected to the seat (410) to be rotatable about the axis.

5. The obstetric and gynecologic diagnosis apparatus (1) of claim 4, wherein, in a first diagnosis state, the upper body support (420) is arranged on a same plane as the seat (410), and the leg cradle (430) is inclined at an angle of 120° to 180° clockwise with respect to the seat.

6. The obstetric and gynecologic diagnosis apparatus (1) of claim 4, wherein, in a second diagnosis state, the upper body support (420), the seat (410), and the leg cradle (430) are arranged on one plane.

7. The obstetric and gynecologic diagnosis apparatus (1) of claim 6, wherein the leg cradle (430) comprises a first cradle (431) and a second cradle (432) on which both legs of the object are respectively placed, and the first cradle (431) and the second cradle (432) are arranged such that one end of the first cradle (431) is spaced apart from one end of the second cradle (432).

8. The obstetric and gynecologic diagnosis apparatus (1) of claim 4, wherein, in a third diagnosis state, the upper body support (420) is inclined at an angle of 110° to 170° counter-clockwise with respect to the seat (410), and the leg cradle (430) is arranged on a same plane as the seat (410).

9. A method of operating the apparatus (1) of claim 1, the method comprising:
obtaining first body information of an object including a gestation period of the object;
obtaining second body information of the object by using a sensor (41);
generating a control signal for a chair unit (400) comprising upper a body support, a seat (410), and a leg cradle (430), based on a stage of the gestation period of the object included in the first body information;
changing a relative position of the upper body support (420), the seat (410), and the leg cradle (430) according to the control signal;
inputting diagnosis completion information; and
changing the relative position of the upper body support (420), the seat (410), and the leg cradle (430) to the initial state;
wherein the second body information of the object is at least one of a weight, a body temperature, a sitting state, and a sitting duration time,
wherein the control signal for the chair unit (400) is generated based on the obtained first body information of the object and the obtained second body information of the object.

10. The method of claim 9, further comprising inputting identification (ID) information of the object,
wherein the first body information of the object is obtained based on the ID information of the object, and the ID information of the object is at least one of name information of the object, fingerprint information of the object, face information of the object, and an ID code corresponding to the object.

11. The method of claim 9, wherein
the diagnosis state is one of first through third diagnosis states, wherein,
in the first diagnosis state, the upper body support (420) is arranged on a same plane as the seat (410), and the leg cradle (430) is inclined at an angle of 120° to 180° clockwise with respect to the seat (410),
in the second diagnosis state, the upper body support (420), the seat (410), and the leg cradle (430) are arranged on one plane, and
in the third diagnosis state, the upper body support (420) is inclined at an angle of 110° to 170° counterclockwise with respect to the seat (410), and the leg cradle (430) is arranged on a same plane as the seat.

12. The method of claim 9, further comprising adjusting the diagnosis state of the chair unit (400) by using an input signal generated by a user.

## Patentansprüche

1. Vorrichtung (1) zur geburtshilflichen und gynäkologischen Diagnose, die Folgendes umfasst:
eine Stuhleinheit (400), auf der ein Objekt zu platzieren ist, wobei die Stuhleinheit (400) eine Oberkörperstütze (420), einen Sitz (410) und eine Beinhalterung (430) umfasst, die nacheinander in einer Richtung angeordnet und miteinander verbunden sind;
eine Steuerung (320), die so konfiguriert ist, dass sie ein Steuersignal zum Bewegen zumindest eines von der Oberkörperstütze (420), dem Sitz (410) und der Beinhalterung (430) gemäß erster Körperinformationen des Objekts einschließlich eines Schwangerschaftszeitraums des Objekts erzeugt;
einen Treiber (50), der so konfiguriert ist, dass er eine Antriebskraft zum Bewegen zumindest eines von der Oberkörperstütze (420), dem Sitz (410) und der Beinhalterung (430) gemäß dem Steuersignal erzeugt; und
zumindest einen Sensor (41), der so konfiguriert ist, dass er zweite Körperinformationen des Objekts erfasst, wobei es sich bei den zweiten Körperinformationen um zumindest eines von einem Gewicht, einer Körpertemperatur, einem Sitzzustand und einer Sitzdauer handelt;
wobei eine relative Position der Oberkörperstütze (420), des Sitzes (410) und der Beinhalterung (430) gemäß einem Stadium des Schwangerschaftszeitraums des Objekts, der in den ersten Körperinformationen enthalten ist, geändert wird,
wobei die Steuerung (320) das Steuersignal zum Bewegen von zumindest einem von der Oberkörperstütze (420), dem Sitz (410) und der Beinhalterung (430) gemäß den ersten Körperinformationen des Objekts und den zweiten Körperinformationen des Objekts erzeugt.

2. Vorrichtung (1) zur geburtshilflichen und gynäkologischen Diagnose nach Anspruch 1, die ferner Folgendes umfasst:
einen Speicher (350), der zum Speichern der ersten Körperinformationen des Objekts konfiguriert ist; und
eine Eingabeschnittstelle (370), die zum Eingeben von Identifizierungsinformationen (ID) des Objekts konfiguriert ist,
wobei die ersten Körperinformationen des Objekts durch die ID-Informationen des Objekts identifiziert werden und es sich bei den ID-Informationen des Objekts um zumindest eine der folgenden Informationen handelt: Namensinformationen des Objekts, Fingerabdruckinformationen des Objekts, Gesichtsinformationen des Objekts und einen dem Objekt entsprechenden ID-Code.

3. Vorrichtung (1) zur geburtshilflichen und gynäkologischen Diagnose nach Anspruch 2, wobei Benutzer-ID-Informationen einschließlich der Informationen eines Benutzers in die Eingabeschnittstelle (370) eingegeben werden, und die Steuerung (320) ein Steuersignal zum Bewegen von zumindest einem von der Oberkörperstütze (420), dem Sitz (410) und der Beinhalterung (430) gemäß den ersten Körperinformationen des Objekts und den Informationen des Benutzers erzeugt.

4. Vorrichtung (1) zur geburtshilflichen und gynäkologischen Diagnose nach Anspruch 1, wobei die Stuhleinheit (400) so angeordnet ist, dass sie in Bezug auf den Boden vertikal beweglich ist, die Oberkörperstütze (420) so mit dem Sitz (410) verbunden ist, dass sie sich um eine Achse drehen kann, und die Beinhalterung (430) so mit dem Sitz (410) verbunden ist, dass sie sich um die Achse drehen kann.

5. Vorrichtung (1) zur geburtshilflichen und gynäkologischen Diagnose nach Anspruch 4, wobei in einem ersten Diagnosezustand die Oberkörperstütze (420) in derselben Ebene wie der Sitz (410) angeordnet ist und die Beinhalterung (430) in einem Winkel von 120° bis 180° im Uhrzeigersinn in Bezug auf den Sitz geneigt ist.

6. Vorrichtung (1) zur geburtshilflichen und gynäkologischen Diagnose nach Anspruch 4, wobei in einem zweiten Diagnosezustand die Oberkörperstütze (420), der Sitz (410) und die Beinhalterung (430) in einer Ebene angeordnet sind.

7. Vorrichtung (1) zur geburtshilflichen und gynäkologischen Diagnose nach Anspruch 6, wobei die Beinhalterung (430) eine erste Halterung (431) und eine zweite Halterung (432) umfasst, auf denen die beiden Beine des Objekts jeweils platziert sind, und die erste Halterung (431) und die zweite Halterung (432) so angeordnet sind, dass ein Ende der ersten Halterung (431) von einem Ende der zweiten Halterung (432) beabstandet ist.

8. Vorrichtung (1) zur geburtshilflichen und gynäkologischen Diagnose nach Anspruch 4, wobei in einem dritten Diagnosezustand die Oberkörperstütze (420) in einem Winkel von 110° bis 170° gegen den Uhrzeigersinn in Bezug auf den Sitz (410) geneigt ist und die Beinhalterung (430) in einer gleichen Ebene wie der Sitz (410) angeordnet ist.

9. Verfahren zum Betreiben der Vorrichtung (1) nach Anspruch 1, wobei das Verfahren Folgendes umfasst:
Erhalten von ersten Körperinformationen eines Objekts einschließlich eines Schwangerschaftszeitraums des Objekts;
Erhalten zweiter Körperinformationen des Objekts unter Verwendung eines Sensors (41);
Erzeugen eines Steuersignals für eine Stuhleinheit (400), die eine Oberkörperstütze, einen Sitz (410) und eine Beinhalterung (430) umfasst, basierend auf einem Stadium des Schwangerschaftszeitraums des Objekts, der in den ersten Körperinformationen enthalten ist;
Ändern einer relativen Position der Oberkörperstütze (420), des Sitzes (410) und der Beinhalterung (430) gemäß dem Steuersignal;
Eingeben von Diagnosebeendigungsinformationen; und
Ändern der relativen Position der Oberkörperstütze (420), des Sitzes (410) und der Beinhalterung (430) in den Ausgangszustand;
wobei es sich bei den zweiten Körperinformationen des Objekts um zumindest eines von einem Gewicht, einer Körpertemperatur, einem Sitzzustand und einer Sitzdauer handelt,
wobei das Steuersignal für die Stuhleinheit (400) basierend auf den erhaltenen ersten Körperinformationen des Objekts und den erhaltenen zweiten Körperinformationen des Objekts erzeugt wird.

10. Verfahren nach Anspruch 9, das ferner das Eingeben von Identifizierungsinformationen (ID-Informationen) des Objekts umfasst,
wobei die ersten Körperinformationen des Objekts basierend auf den ID-Informationen des Objekts erhalten werden und es sich bei den ID-Informationen des Objekts um zumindest eine der folgenden Informationen handelt: Namensinformationen des Objekts, Fingerabdruckinformationen des Objekts, Gesichtsinformationen des Objekts und einen dem Objekt entsprechenden ID-Code.

11. Verfahren nach Anspruch 9, wobei es sich bei dem Diagnosezustand um einen von einem ersten bis dritten Diagnosezustand handelt, wobei
in dem ersten Diagnosezustand die Oberkörperstütze (420) in derselben Ebene wie der Sitz (410) angeordnet ist und die Beinhalterung (430) in einem Winkel von 120° bis 180° im Uhrzeigersinn in Bezug auf den Sitz (410) geneigt ist,
in dem zweiten Diagnosezustand die Oberkörperstütze (420), der Sitz (410) und die Beinhalterung (430) in einer Ebene angeordnet sind, und
in dem dritten Diagnosezustand die Oberkörperstütze (420) in einem Winkel von 110° bis 170° gegen den Uhrzeigersinn in Bezug auf den Sitz (410) geneigt ist und die Beinhalterung (430) in einer gleichen Ebene wie der Sitz angeordnet ist.

12. Verfahren nach Anspruch 9, das ferner das Anpassen des Diagnosezustands der Stuhleinheit (400) unter Verwendung eines von einem Benutzer erzeugten Eingabesignals umfasst.

## Revendications

1. Dispositif de diagnostic obstétrical et gynécologique (1) comprenant :
une unité formant chaise (400) sur laquelle un objet est à placer, l'unité formant chaise (400) comprenant un support de partie supérieure du corps (420), un siège (410) et un repose-jambes (430) agencés les uns à la suite des autres dans une direction et reliés entre eux,
un organe de commande (320) conçu pour générer un signal de commande visant à déplacer le support de partie supérieure du corps (420), le siège (410) et/ou le repose-jambes (430) en fonction de premières informations corporelles de l'objet, dont la période de gestation de l'objet,
un dispositif moteur (50) conçu pour générer une force motrice permettant de déplacer le support de partie supérieure du corps (420), le siège (410) et/ou le repose-jambes (430) conformément au signal de commande, et
au moins un capteur (41) conçu pour détecter des deuxièmes informations corporelles de l'objet, les deuxièmes informations corporelles concernant le poids, la température corporelle, l'état assis et/ou la durée en position assise ;
une position relative du support de partie supérieure du corps (420), du siège (410) et du repose-jambes (430) étant modifiée en fonction d'un stade de la période de gestation de l'objet incluse dans les premières informations corporelles,
ledit organe de commande (320) générant le signal de commande visant à déplacer le support de partie supérieure du corps (420), le siège (410) et/ou le repose-jambes (430) en fonction des premières informations corporelles de l'objet et des deuxièmes informations corporelles de l'objet.

2. Dispositif de diagnostic obstétrical et gynécologique (1) selon la revendication 1, comprenant en outre :
un dispositif de stockage (350) conçu pour le stockage des premières informations corporelles de l'objet, et
une interface d'entrée (370) conçue pour la saisie d'informations d'identification de l'objet ;
lesdites premières informations corporelles de l'objet étant identifiées par les informations d'identification de l'objet, et les informations d'identification de l'objet consistant en des informations relatives au nom de l'objet, des informations relatives aux empreintes digitales de l'objet, des informations relatives au visage de l'objet et/ou un code d'identification correspondant à l'objet.

3. Dispositif de diagnostic obstétrical et gynécologique (1) selon la revendication 2, dans lequel des informations d'identification de l'utilisateur, dont des informations relatives à un utilisateur, sont saisies dans l'interface d'entrée (370), et l'organe de commande (320) génère un signal de commande visant à déplacer le support de partie supérieure du corps (420), le siège (410) et/ou le repose-jambes (430) en fonction des premières informations corporelles de l'objet et des informations relatives à l'utilisateur.

4. Dispositif de diagnostic obstétrical et gynécologique (1) selon la revendication 1, dans lequel l'unité formant chaise (400) est conçue pour être mobile verticalement par rapport au sol, le support de partie supérieure du corps (420) est relié au siège (410) en étant rotatif sur un axe, et le repose-jambes (430) est relié au siège (410) en étant rotatif sur ledit axe.

5. Dispositif de diagnostic obstétrical et gynécologique (1) selon la revendication 4, dans lequel, dans un premier état de diagnostic, le support de partie supérieure du corps (420) est agencé sur le même plan que le siège (410), et le repose-jambes (430) est incliné à un angle allant de 120° à 180° dans le sens des aiguilles d'une montre par rapport au siège.

6. Dispositif de diagnostic obstétrical et gynécologique (1) selon la revendication 4, dans lequel, dans un deuxième état de diagnostic, le support de partie supérieure du corps (420), le siège (410) et le repose-jambes (430) sont agencés sur un même plan.

7. Dispositif de diagnostic obstétrical et gynécologique (1) selon la revendication 6, dans lequel le repose-jambes (430) comprend un premier repose-jambes (431) et un deuxième repose-jambes (432) sur lesquels les deux jambes de l'objet sont respectivement placées, et le premier repose-jambes (431) et le deuxième repose-jambes (432) sont agencés de façon qu'une extrémité du premier repose-jambes (431) est espacée d'une extrémité du deuxième repose-jambes (432).

8. Dispositif de diagnostic obstétrical et gynécologique (1) selon la revendication 4, dans lequel, dans un troisième état de diagnostic, le support de partie supérieure du corps (420) est incliné à un angle allant de 110° à 170° dans le sens contraire des aiguilles d'une montre par rapport au siège (410), et le repose-jambes (430) est agencé sur le même plan que le siège (410).

9. Procédé d'exploitation du dispositif (1) selon la revendication 1, le procédé comprenant les étapes consistant à :
obtenir des premières informations corporelles d'un objet, dont la période de gestation,
obtenir des deuxièmes informations corporelles de l'objet à l'aide d'un capteur (41) ;
générer un signal de commande destiné à une unité formant chaise (400) comprenant un support de partie supérieure du corps, un siège (410) et un repose-jambes (430), compte tenu d'un stade de la période de gestation de l'objet incluse dans les premières informations corporelles,
modifier une position relative du support de partie supérieure du corps (420), du siège (410) et du repose-jambes (430) en fonction du signal de commande,
saisir des informations d'achèvement de diagnostic, et
modifier la position relative du support de partie supérieure du corps (420), du siège (410) et du repose-jambes (430) pour revenir à l'état initial,
lesdites deuxièmes informations corporelles de l'objet concernant le poids, la température corporelle, l'état assis et/ou la durée en position assise,
ledit signal de commande destiné à l'unité formant chaise (400) étant généré en fonction des premières informations corporelles de l'objet obtenues et des deuxièmes informations corporelles de l'objet obtenues.

10. Procédé selon la revendication 9, comprenant en outre la saisie d'informations d'identification de l'objet,
lesdites premières informations corporelles de l'objet étant obtenues en fonction des informations d'identification de l'objet, et les informations d'identification de l'objet consistant en des informations relatives au nom de l'objet, des informations relatives aux empreintes digitales de l'objet, des informations relatives au visage de l'objet et/ou un code d'identification correspondant à l'objet.

11. Procédé selon la revendication 9, dans lequel l'état de diagnostic est un premier, deuxième ou troisième état de diagnostic, étant entendu que
dans le premier état de diagnostic, le support de partie supérieure du corps (420) est agencé sur le même plan que le siège (410), et le repose-jambes (430) est incliné à un angle allant de 120° à 180° dans le sens des aiguilles d'une montre par rapport au siège (410),
dans le deuxième état de diagnostic, le support de partie supérieure du corps (420), le siège (410) et le repose-jambes (430) sont agencés sur un même plan, et
dans le troisième état de diagnostic, le support de partie supérieure du corps (420) est incliné à un angle allant de 110° à 170° dans le sens contraire des aiguilles d'une montre par rapport au siège (410), et le repose-jambes (430) est agencé sur le même plan que le siège.

12. Procédé selon la revendication 9, comprenant en outre le réglage de l'état de diagnostic de l'unité formant chaise (400) à l'aide d'un signal d'entrée généré par un utilisateur.
